(19) Europäisches Patentamt / European Patent Office / Office européen des brevets

(11) **EP 3 408 612 B1**

(12) **FASCICULE DE BREVET EUROPEEN**

(45) Date de publication et mention de la délivrance du brevet:
**14.07.2021  Bulletin 2021/28**

(21) Numéro de dépôt: **17706285.8**

(22) Date de dépôt: **24.01.2017**

(51) Int Cl.:
*G01C 9/00* *(2006.01)*      *A61B 5/11* *(2006.01)*
*G01C 21/12* *(2006.01)*      *A61B 5/00* *(2006.01)*
*A61B 5/22* *(2006.01)*

(86) Numéro de dépôt international:
**PCT/FR2017/050147**

(87) Numéro de publication internationale:
**WO 2017/129890 (03.08.2017 Gazette 2017/31)**

(54) **PROCEDE D'ESTIMATION DE L'ACTIVITE PHYSIQUE D'UN MEMBRE SUPERIEUR**

VERFAHREN ZUR KALKULATION DER PHYSISCHEN AKTIVITÄT EINER OBEREN GLIEDMASSE

METHOD FOR ESTIMATING THE PHYSICAL ACTIVITY OF AN UPPER LIMB

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.01.2016  FR 1650652**

(43) Date de publication de la demande:
**05.12.2018  Bulletin 2018/49**

(73) Titulaires:
- **SYSNAV**
  **27200 Vernon (FR)**
- **Association Institut de Myologie**
  **75013 Paris (FR)**

(72) Inventeurs:
- **GRELET, Marc**
  **27200 Vernon (FR)**
- **DORVEAUX, Eric**
  **27200 Vernon (FR)**
- **VISSIERE, David**
  **75009 Paris (FR)**
- **SERVAIS, Laurent**
  **92160 Antony (FR)**
- **HOGREL, Jean- Yves**
  **92120 Montrouge (FR)**
- **MORAUX, Amélie**
  **27200 Vernon (FR)**

(74) Mandataire: **Regimbeau**
  **20, rue de Chazelles**
  **75847 Paris Cedex 17 (FR)**

(56) Documents cités:
**EP-A1- 2 796 123     US-A1- 2015 374 283**

- **Hendrik Johannes Luinge: "Inertial sensing of human movement", , 1 janvier 2002 (2002-01-01), XP055335532, Extrait de l'Internet: URL:https://www.xsens.com/images/stories/PDF/Inertial_Sensing_of_Human_Movement_Thesis_Luinge.pdf [extrait le 2017-01-16]**

EP 3 408 612 B1

**Description**

DOMAINE TECHNIQUE GENERAL

La présente invention concerne le domaine de la mesure d'activité physique du corps humain.

Plus précisément, elle concerne un procédé d'estimation de l'activité physique d'un membre supérieur d'une personne via des techniques magnéto-inertielles.

ETAT DE L'ART

De nombreux dispositifs sont proposés en vente pour mesurer l'activité physique d'une personne en utilisant des capteurs sensibles à certains types de mouvements. Un exemple existant depuis de nombreuses années est le podomètre, dont le principe est de compter le nombre de pas effectués par le porteur.

Plus récemment, des dispositifs ont été développés fournissant des informations plus développées sur l'activité physique que le simple comptage des pas. On peut par exemple souhaiter distinguer marche et course ou bien connaître le type de marche et l'effort associé. Au-delà de l'analyse de la marche, certains dispositifs visent à prendre en compte l'ensemble des mouvements effectués par la personne pour par exemple estimer la quantité d'énergie utilisée lors d'une activité. Pour cela, un ou plusieurs objets sont portés par la personne. De nombreuses configurations ont été proposées concernant le port de ces objets. Par exemple, ils peuvent être portés au poignet comme une montre, placés dans une poche ou à la ceinture, accrochés au torse, à une jambe, à une chaussure, etc.

Mesurer l'activité physique d'une personne a des applications variées. De nombreuses personnes souhaitent par exemple augmenter ou réguler leurs dépenses caloriques notamment pour le contrôle de la masse corporelle. Dans ce cadre, il peut être utile d'estimer l'activité physique effectuée par ces personnes et de réaliser un suivi en continu dans le temps. Certains sportifs peuvent chercher à optimiser leur entraînement ou simplement à suivre l'évolution de leur performance. Et indépendamment d'un objectif particulier, chacun peut s'intéresser à son niveau de condition physique. Enfin dans un cadre médical, il peut être essentiel d'estimer et de suivre l'évolution de l'activité physique d'un patient. Pour des pathologies associées à des difficultés musculaires par exemple, la mesure de l'activité physique est un indicateur de l'état du patient. Le médecin peut alors suivre l'évolution de la maladie et évaluer l'efficacité d'un traitement. Cela peut servir à démontrer la validité d'un traitement ou à l'adapter en fonction de la réponse du patient.

Pour la mesure des mouvements, quelques capteurs fréquemment utilisés dans les dispositifs existants sont les capteurs d'accélération, de vitesse angulaire, de champ magnétique, de pression atmosphérique, de position GPS, etc. En outre des mesures physiologiques sont parfois effectuées comme par exemple les pulsations cardiaques ou la température corporelle.

Les premiers podomètres utilisaient ainsi un dispositif mécanique sensible aux mouvements créés lors de chaque pas. La plupart des dispositifs modernes intègrent cependant des capteurs disposant d'interfaces électroniques et souvent de type MEMS. Les composants MEMS sont issus de développements récents et leur utilisation est largement répandue dans l'industrie électronique grand public. Les avantages de ces capteurs sont le faible encombrement et une consommation électrique réduite ce qui permet de les intégrer au sein d'objets portés par une personne sans causer de gêne particulière. De plus ces composants sont disponibles à des coûts compatibles avec l'électronique grand public et très inférieurs à ceux de composants tactiques.

Une difficulté avec de tels dispositifs concerne la fiabilité des mesures effectuées. De nombreux indicateurs sont donnés mais leur calcul n'est souvent pas documenté. Et même si la méthode de calcul est expliquée, la représentativité de la variable n'est pas connue. Même pour des variables simples comme le comptage de pas, la performance est difficile à évaluer car le lien avec un effort physique n'est pas direct, le pas d'un piétinement n'a rien à voir celui d'une course en sprint.

On connait des méthodes pour calculer de façon nettement plus précise le déplacement relatif par intégration des capteurs d'une centrale inertielle. Une centrale inertielle est constituée au minimum de trois accéléromètres et de trois gyromètres disposés en triaxe. Typiquement, les gyromètres « maintiennent » un repère, dans lequel une double intégration temporelle des mesures des accéléromètres permet d'estimer le mouvement. Cependant les centrales inertielles ou magnéto-inertielles nécessaires pour cette technique sont lourdes et chères telles celles embarquées dans un avion ou un sous-marin et ne peuvent pas être portées par une personne. Avec des capteurs miniaturisés de type MEMS, la performance permet de calculer un mouvement relatif pendant une durée maximale de quelques secondes.

Pour cette raison des méthodes complémentaires ou alternatives ont été proposées pour se repérer en dépit de la performance limitée des capteurs. Il s'agit alors d'utiliser l'information de capteurs additionnels, on peut citer par exemple la mesure du gradient de champ magnétique pour estimer la vitesse ou l'utilisation des caméras vidéos pour s'orienter par rapport à l'environnement. L'orientation par rapport à la direction verticale peut s'obtenir par la mesure du champ de gravité ce dispositif est parfois appelé inclinomètre. Le cap peut s'obtenir par la mesure du champ de gravité terrestre.

On constate cependant que ces mesures restent bruitées et mal exploitées.

La demande FR1559591 a ainsi proposé un procédé d'estimation du mouvement d'un piéton en marche grâce à des moyens de mesure inertielle solidaires d'un membre inférieur dudit piéton.

La demande US 2015/374283 divulgue un procédé d'estimation de l'activité physique exercée par un membre supérieur d'une personne, le procédé étant caractérisé en ce qu'il comprend des étapes de : acquisition par des moyens de mesure inertielle solidaires d'un avant-bras dudit membre supérieur de ladite personne d'une vitesse angulaire dudit avant-bras ; et estimation par des moyens de traitement de données d'un couple exercé par les muscles d'un bras du membre supérieur sur ledit avant-bras en fonction de la vitesse angulaire mesurées. La demande EP2796123 divulgue l'estimation d'un couple exercé par la jambe d'une personne.

Il serait souhaitable de disposer d'une méthode similaire pour estimer les efforts physiques réalisés par cette fois par un membre supérieur, et c'est l'objet de la présente invention.

PRESENTATION DE L'INVENTION

La présente invention se rapporte ainsi selon un premier aspect à un procédé d'estimation de l'activité physique exercée par un membre supérieur d'une personne selon la revendication 1.

Selon d'autres caractéristiques avantageuses et non limitatives :

- Le procédé comprend suite à l'étape (a) une étape (a1) de détermination de ladite orientation de l'avant-bras ;
- l'étape (a) comprend en outre l'acquisition par les moyens de mesure inertielle d'une accélération spécifique dudit membre supérieur, l'orientation de l'avant-bras étant déterminée en fonction et l'accélération et la vitesse angulaire mesurées ;
- la détermination de l'orientation de l'avant-bras comprend la mise en œuvre d'un filtre estimateur d'état linéaire ou non linéaire ;
- lesdits moyens de mesure inertielle sont disposés sur ledit avant-bras entre un coude et un poignet ;
- les paramètres physiques de l'avant-bras comprennent une longueur de l'avant-bras et un rayon de l'avant-bras ;
- le couple estimé à l'étape (b) est un couple par unité de masse dudit avant-bras donné par la formule

$$\overrightarrow{C_M} = \begin{pmatrix} \frac{R^2}{4} + \frac{L^2}{3} & 0 & 0 \\ 0 & \frac{R^2}{4} + \frac{L^2}{3} & 0 \\ 0 & 0 & \frac{R^2}{2} \end{pmatrix} \times \begin{bmatrix} \frac{d\omega_x}{dt} \\ \frac{d\omega_y}{dt} \\ \frac{d\omega_z}{dt} \end{bmatrix} - \frac{L}{2} \, g \, \cos \theta_e \, \overrightarrow{e_n},$$

  où $\vec{\omega}$ est la vitesse angulaire mesurée, $R$ le rayon de l'avant-bras, L la longueur de l'avant-bras, $\vec{g}$ l'accélération de la pesanteur, $\theta_e$ l'angle d'élévation de l'avant-bras et $\overrightarrow{e_n}$ le vecteur unitaire normal à l'avant-bras et à la direction de l'accélération de la pesanteur ;
- la puissance exercée par le membre supérieur est déterminée à l'étape (c) par le produit scalaire du couple estimé et de la vitesse angulaire mesurée ;
- l'étape (c) comprend l'identification du caractère moteur ou de retenue de la puissance déterminée ;
- le procédé est un procédé d'estimation d'une grandeur représentative de l'activité physique exercée par le membre supérieur de la personne, le procédé comprenant une étape (d) d'estimation de ladite grandeur physique à partir de la puissance déterminée ;
- ladite grandeur physique est l'énergie exercée par le membre supérieur sur un intervalle de temps, estimée à l'étape (d) en sommant la valeur absolue des puissances déterminées à chaque instant dudit intervalle de temps ;
- le procédé comprend en outre une étape (e) de comparaison de ladite grandeur physique estimée avec au moins un seuil prédéterminé de sorte à identifier un problème musculaire du membre supérieur de la personne.

Selon un deuxième aspect, l'invention concerne un équipement de l'activité physique exercée par un membre supérieur d'une personne selon la revendication 12.

Selon d'autres caractéristiques avantageuses et non limitatives :

- L'équipement est un boitier comprenant les moyens de mesure inertielle ;
- L'équipement comprend en outre des moyens d'attache du boitier au membre supérieur, et des moyens de communication.
- L'équipement est un terminal mobile ou un serveur, adapté pour communiquer avec un boitier comprenant les moyens de mesure inertielle.

Selon un quatrième et un cinquième aspect, l'invention concerne un produit programme d'ordinateur selon la revendication 14; et un moyen de stockage lisible par un équipement informatique selon la revendication 15.

PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :

- la figure 1 est un schéma d'équipements pour la mise en œuvre du procédé selon l'invention ;
- La figure 2 représente plus en détail un exemple de boitier pour la mise en œuvre du procédé selon l'invention ;
- la figure 3 représente schématiquement un membre supérieur équipé d'un boitier pour la mise en œuvre du procédé selon l'invention.

DESCRIPTION DETAILLEE

*Architecture*

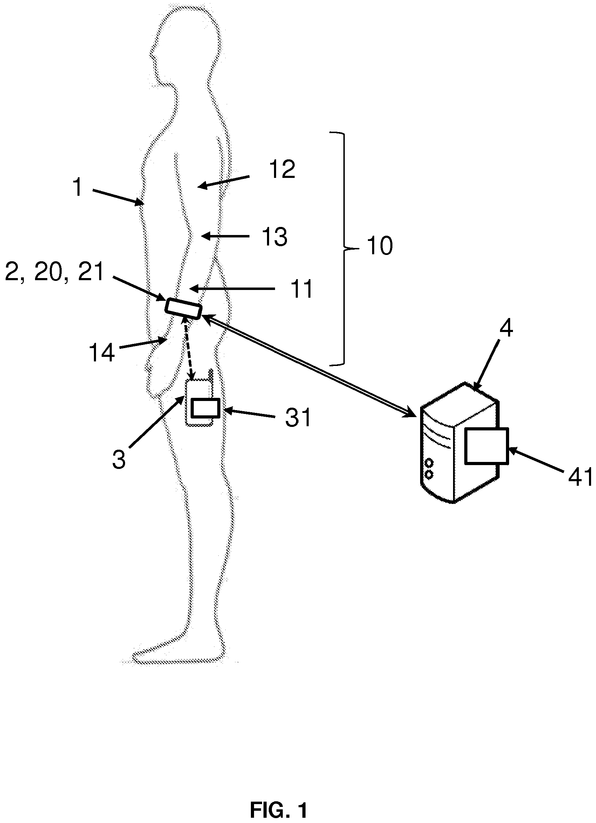

En référence à la **figure 1,** le présent procédé permet l'estimation de l'activité physique exercée par un membre supérieur 10 d'une personne 1.

Par « activité physique », on entend une grandeur physique représentative des efforts exercés par les muscles du membre supérieur, la grandeur physique étant choisie parmi un ensemble de grandeurs mécaniques telles que la puissance instantanée, la puissance moyenne, le travail, l'énergie, etc. Ces notions seront explicitées plus loin. De façon générale, le présent procédé peut être défini comme un procédé d'estimation d'au moins une grandeur physique représentative de l'activité physique exercée par un membre supérieur 10 d'une personne 1.

La personne 1 présente au moins un membre supérieur 10 (i.e. un « bras » dans le langage courant, même si comme on le verra plus bas cette appellation est impropre, un bras désignant anatomiquement « l'arrière-bras », c'est-à-dire la partie entre l'épaule et le coude) équipée de moyens de mesure inertielle 20. On comprendra que chacun des deux membres supérieurs 10 de la personne 1 peut être équipé de moyens de mesure inertielle 20.

Les membres supérieurs 10 sont chacun constitués de trois segments : le bras 12, l'avant-bras 11 et la main. L'articulation du coude 13 relie le bras à l'avant-bras, et le poignet 14 relie l'avant-bras à la main.

Plus précisément, les moyens de mesure inertielle 20 sont solidaires de l'avant-bras 11, i.e. qu'ils présentent un mouvement sensiblement identique dans le référentiel terrestre, on verra comment plus loin. Les moyens de mesure inertielle sont plus précisément disposés entre le coude 13 et le poignet 14 de la personne (inclus), et de façon générale tout emplacement présentant par rapport à au coude 13 du membre supérieur 10 sensiblement uniquement un mouvement de rotation, c'est à dire dû à un bras de levier.

La distance entre le coude 13 et les moyens 20 importe peu comme le verra, la personne pouvant le fixer à proximité du poignet 14 pour plus de distance entre le coude 13 et les moyens 20 et donc plus de précision, mais quelle que soit sa position le présent procédé fonctionne.

Comme on le verra plus loin, des paramètres physiques dudit avant-bras 11 sont nécessaires, en particulier des paramètres anthropométriques dont une longueur de l'avant-bras 11 et un rayon de l'avant-bras 11.

Ces grandeurs peuvent être des paramètres d'entrée (saisis par la personne 1) ou alors une valeur moyenne peut être utilisée. On note que le rayon intervient dans le calcul mais comme l'on verra il peut être négligé car le terme lié au rayon est petit par rapport aux autres termes.

Par ailleurs la masse de l'avant-bras 11 peut être utilisée, mais il est possible de fournir des valeurs normalisées des grandeurs représentatives de l'activité physique (i.e. par unité de masse de l'avant-bras 11), ce qui fait que sa connaissance est optionnelle.

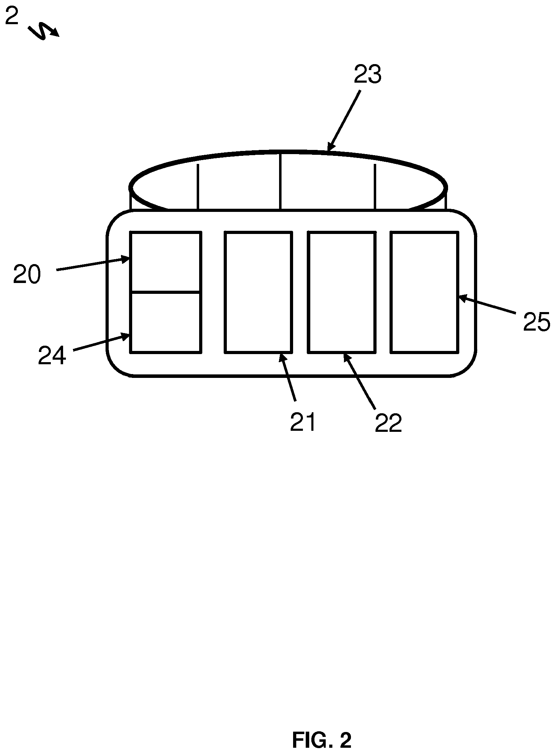

Les moyens de mesure inertielle 20 sont préférentiellement ceux d'un boitier 2 tel que représenté sur la **figure 2** présentant des moyens 23 d'attache au membre supérieur 10. Ces moyens d'attache 23 consistent par exemple en un bracelet par exemple à bande autoagrippante qui enserre le membre et permet la liaison solidaire. Comme l'on verra plus tard, il est en effet souhaitable que les moyens de mesure inertielle 20 soient disposés le plus près possible du poignet 14, et si possible ne puissent pas se déplacer le long du membre 10.

Par moyens de mesure inertielle 20, on entend une centrale à inertie comprenant au moins trois accéléromètres et trois gyromètres disposés en triaxe. Les gyromètres mesurent la vitesse angulaire instantanée de la centrale inertielle par rapport au référentiel terrestre, notée $\vec{\omega}$. Les accéléromètres sont sensibles aux forces extérieures autres que gravitationnelles appliquées sur le capteur, et permettent de mesurer une accélération notée $\vec{\gamma}$. Comme on le verra, les moyens 20 peuvent comprendre des moyens 24 d'acquisition de l'orientation (dont la vitesse angulaire est la dérivée) voire de manière plus limitée seulement de l'angle d'élévation (i.e. une seule composante de l'orientation) de l'avant-bras 11. Par exemple un inclinomètre qui est souvent un simple accéléromètre ne permet pas d'estimer l'orientation ni la vitesse angulaire de l'avant-bras 11 mais seulement l'angle d'élévation et avec des erreurs plus importantes qu'avec une centrale inertielle complète. Il est à noter que dans un mode de réalisation où seulement l'angle d'élévation est mesuré grâce à un simple inclinomètre, cette donnée peut être « débruitée » grâce aux mesures de vitesse angulaire des moyens 20. On comprend toutefois que l'orientation complète peut être mesurée par des moyens 24 adaptés, par exemple optiques ou magnétiques. Comme on le verra les moyens 24 (qu'ils mesurent une composante ou toute l'orientation) ne sont pas indispensables et seuls des moyens de mesure inertielle de la vitesse angulaire sont essentiels.

Le boitier 2 peut comprendre des moyens de traitement 21 (typiquement un processeur) pour la mise en œuvre directement en temps réel des traitements du présent procédé, ou bien les mesures peuvent être émises via des moyens de communication 25 vers un dispositif externe tel qu'un terminal mobile (smartphone) 3, voire un serveur distant 4, ou encore les mesures peuvent être enregistrées dans des moyens de stockage de données 22 locaux (une mémoire par exemple de type flash) mémoire locale pour un traitement a posteriori par exemple sur le serveur 4.

Les moyens de communication 25 peuvent mettre en œuvre une communication sans fil à courte portée par exemple Bluetooth ou Wifi (en particulier dans un mode de réalisation avec un terminal mobile 3) voire être des moyens de connexion à un réseau mobile (typiquement UMTS/LTE) pour une communication à longue distance. Il est à noter que les moyens de communication 25 peuvent être par exemple une connectique filaire (typiquement USB) pour transférer les données des moyens de stockage de données 22 locaux à ceux d'un terminal mobile 3 ou d'un serveur 4.

Si c'est un terminal mobile 3 (respectivement un serveur 4) qui héberge « l'intelligence », il comprend des moyens de traitement 31 (respectivement 41) tels qu'un processeur pour la mise en œuvre des traitements du présent procédé qui vont être décrits. Lorsque les moyens de traitement utilisés sont ceux 21 du boitier 2, celui-ci peut encore inclure des moyens de communication 25 pour transmettre la position estimée. Par exemple la position du porteur peut être envoyée au terminal mobile 3 pour afficher la position dans une interface d'un logiciel de navigation.

Dans la suite de la présente description, on verra que les moyens de traitement de données 21, 31, 41 respectivement du boitier 2, d'un smartphone 3 et d'un serveur distant 4 peuvent indifféremment et selon les applications réaliser tout ou partie des étapes du procédé.

*Principe et notations*

Dans une première étape (a), le procédé comprend l'acquisition par les moyens de mesure inertielle 20 d'au moins la vitesse angulaire $\vec{\omega}$ dudit membre supérieur 10, et plus précisément de l'avant-bras 11.

De façon préférée, l'accélération spécifique $\vec{\gamma}$ de l'avant-bras 11 est également mesurée. Avantageusement, le procédé comprend suite à l'étape (a) une étape (a1) de détermination par les moyens de traitement de données 21, 31, 41 de ladite orientation de l'avant-bras 11, et ce grâce à cette accélération mesurée (et la vitesse angulaire mesurée).

On note que dans la suite de la description, lorsqu'on mentionne accélération/vitesse/orientation du membre supérieur 10, on entend au niveau des moyens de mesure inertielle 20, car des points du membre supérieur 10 situés par exemple au niveau du bras 12 auront un mouvement différent.

On introduit un référentiel inertiel terrestre $(R_i)$ ainsi qu'un référentiel $(R_b)$ ayant pour origine le centre du boitier 2 et dont les axes sont liés à ceux du boitier 2. La vitesse angulaire $\vec{\omega}$ de l'avant bras 11 est égal à la rotation de $(R_b)$ par rapport à $(R_i)$. L'accélération spécifique $\vec{\gamma}$ est égal à la somme de l'accélération du boitier dans le référentiel inertiel terrestre moins la valeur du champ de gravité $\vec{g}$. Les capteurs inertiels gyromètre et accéléromètre (moyens de mesure inertielle 20) mesurent respectivement $\vec{\omega}$ et $\vec{\gamma}$ exprimés dans le repère $(R_b)$.

Ces grandeurs sont avantageusement mesurées avec un échantillonnage dt (i.e. toutes les « dt » secondes) avec dt très petit devant le temps caractéristique des mouvements de la personne 1, typiquement 10 ms.

L'orientation des moyens 20 par rapport au référentiel inertiel terrestre peut être donnée par exemple par une matrice de rotation (notée R), un quaternion d'attitude (noté q), l'attitude est synonyme d'orientation dans l'espace ou des angles d'Euler (roulis $\varphi$, tangage $\theta$, lacet $\psi$). Ces trois représentations sont équivalentes, on les utilise donc indifféremment dans ce document.

L'initialisation de l'attitude peut se faire par exemple à partir des mesures d'accélération (et le cas échéant des mesures d'un éventuel magnétomètre) en considérant que le membre 10 et donc les moyens 20 sont immobiles au démarrage et que le champ magnétique est égal au champ magnétique terrestre. Dans ce cas l'accélération mesurée est égale à l'opposée du champ gravitationnel $\vec{\gamma} = -\vec{g}$. On a alors le roulis et le tangage avec les formules suivantes:

$$\varphi = -\tan^{-1}\frac{\gamma_y}{\gamma_z}$$

$$\theta = \sin^{-1} \frac{\gamma_x}{\sqrt{\gamma_x{}^2 + \gamma_y{}^2 + \gamma_z{}^2}}$$

Le cap magnétique peut alors se calculer à partir de la mesure de champ magnétique avec la formule:

$$\psi = \tan^{-1} \frac{B_z \cdot \sin\varphi - B_y \cdot \cos\varphi}{B_x \cdot \cos\theta + B_y \cdot \sin\theta \cdot \sin\varphi + B_z \cdot \sin\theta \cdot \cos\varphi}$$

La formule donnant la matrice de passage du référentiel terrestre ($R_i$) au référentiel ($R_b$) des moyens 20 à partir des angles d'Euler est:

$$R_{R_i \rightarrow R_b}$$
$$= \begin{bmatrix} \cos\theta \cdot \cos\psi & -\cos\theta \cdot \sin\psi & \sin\theta \\ \cos\psi \cdot \sin\theta \cdot \sin\varphi + \cos\varphi \cdot \sin\psi & \cos\varphi \cdot \cos\psi - \sin\theta \cdot \sin\varphi \cdot \sin\psi & -\cos\theta \cdot \sin\varphi \\ \sin\varphi \cdot \sin\psi - \cos\varphi \cdot \cos\psi \cdot \sin\theta & \cos\psi \cdot \sin\varphi + \cos\varphi \cdot \sin\theta \cdot \sin\psi & \cos\theta \cdot \cos\varphi \end{bmatrix}$$

La formule ci-avant permet d'estimer l'orientation du boitier 2 à tout moment à partir des mesures d'accélération et de vitesse angulaire mais une limitation importante vient de l'hypothèse d'immobilité ($\vec{\gamma} = -\vec{g}$). En pratique l'avant-bras 11 bouge et l'accélération n'est pas négligeable devant la valeur du champ de gravité. Pour cette raison il est opportun d'utiliser les mesures de vitesse angulaire pour filtrer les erreurs liées aux accélérations subies par le boitier 2.

En d'autres termes, la détermination de l'orientation de l'avant-bras 11 comprend avantageusement la mise en œuvre d'un filtre estimateur d'état linéaire ou non linéaire

Les méthodes permettant d'estimer l'orientation d'une centrale inertielle sont connues. Pour cela, on peut utiliser un filtre estimateur d'état linéaire (filtre Luenberger, filtre de Kalman, etc.) ou non-linéaire (filtre de Kalman étendu, observateur invariant, etc.). Dans la présente description, on rappelle les principales étapes du filtre de Kalman étendu, mais l'homme du métier saura transposer à d'autres filtres.

Dans un filtre de Kalman étendu, l'état est représenté par un vecteur par exemple un quaternion d'attitude de dimension 4. Il est possible d'ajouter d'autres états à ce vecteur pour améliorer l'estimation, par exemple le biais des capteurs. Une matrice de covariance est utilisée pour estimer la covariance entre chaque état du filtre, elle est de dimension $n^2$ donc de dimension 16 dans l'exemple du quaternion d'attitude. Le filtre de Kalman s'effectue en deux étapes, une étape de prédiction et une étape mise à jour. Lors de la mise à jour, un gain de Kalman $K_n$ est calculé à partir de la matrice de covariance.

L'étape de prédiction repose sur un modèle lié à la vitesse angulaire $\vec{\omega}$. On utilise l'équation différentielle sur la matrice de passage $R_{R_0 \rightarrow R}$, et les coordonnées de $\omega$ exprimées dans la base du boitier 2.

$$\dot{R}_{R_i \rightarrow R_b} = \begin{bmatrix} 0 & -\omega_{bz} & \omega_{by} \\ \omega_{bz} & 0 & -\omega_{bx} \\ -\omega_{by} & \omega_{bx} & 0 \end{bmatrix} \times R_{R_i \rightarrow R_b}$$

En considérant que la période de l'échantillonnage notée dt est suffisamment petite, on utilise l'approximation d'un développement limité à l'ordre 1:

$$R_{R_i \rightarrow R_b}(t + dt) = R_{i \rightarrow b}(t) + \dot{R}_{i \rightarrow b}(t)\, dt$$

En notant $\hat{R}_n$ l'estimation de la matrice $R_{R_i \rightarrow R_b}$ après n pas d'échantillons, l'estimation de la matrice $\hat{R}_n$ pour chaque mesure $\vec{\omega_n}$ est:

$$\widehat{R}_{n+1} = \widehat{R}_n + \begin{bmatrix} 0 & -\omega_{n,z} & \omega_{n,y} \\ \omega_{n,z} & 0 & -\omega_{n,x} \\ -\omega_{n,y} & \omega_{n,x} & 0 \end{bmatrix} \times \widehat{R}_n \, dt$$

L'équation est donnée ici pour une matrice de rotation mais une équation différentielle équivalente existe entre quaternion d'attitude et vitesse angulaire. En parallèle, la matrice de covariance est modifiée en linéarisant l'équation différentielle.

L'étape de mise à jour repose sur la mesure de l'accéléromètre. On utilise le fait que l'accélération du boitier 2 est égale à la somme de l'accélération spécifique mesurée par les moyens 20 et du champ de gravité.

$$\vec{a} = \vec{\gamma} + \vec{g}$$

On sait de plus que l'accélération $\vec{a}$ est en moyenne nulle (on comprend que le membre supérieur 10 revient régulièrement à sa position de départ et que les mouvements dans un sens ou l'autre se compensant statistiquement). On en déduit que $\vec{\gamma}$ exprimée dans le repère inertiel terrestre est égale en moyenne à $-\vec{g}$.

A partir de la matrice de rotation $\widehat{R}_n$ et des mesures des moyens 20 dans le repère du boitier 2, on peut exprimer l'accélération spécifique dans le repère inertiel terrestre:

$$\widehat{R}_n^{-1} \begin{bmatrix} \gamma_{n,x} \\ \gamma_{n,y} \\ \gamma_{n,z} \end{bmatrix}$$

La différence entre le champ de gravité estimé et le champ de gravité terrestre réel s'écrit alors:

$$\text{erreur}_n = \widehat{R}_n^{-1} \begin{bmatrix} \gamma_{n,x} \\ \gamma_{n,y} \\ \gamma_{n,z} \end{bmatrix} - \begin{bmatrix} 0 \\ 0 \\ g \end{bmatrix}$$

Cette différence est utilisée pour le recalage de l'attitude lors de l'étape de mise à jour. En linéarisant cette formule, on parvient à calculer le gain de Kalman et par suite de mettre à jour l'état et la matrice de covariance.

La mise à jour de la matrice de covariance repose sur l'hypothèse que les erreurs dues aux capteurs et aux approximations sont modélisées comme un bruit de distribution gaussienne. La variance est estimée en mesurant le bruit des capteurs au repos et à partir d'hypothèses sur les mouvements effectués par l'avant-bras 11.

Il est ainsi possible aux moyens de traitement de données 21, 31, 41 d'estimer l'orientation du boitier 2 (grâce aux mesures d'accélération et de vitesse angulaire des moyens 20) et par conséquent celle de l'avant-bras 11. On comprendre tout autre dispositif 24 permettant cette mesure serait également utilisable, en notant toutefois qu'un inclinomètre seul mesurant l'angle d'élévation ne donne pas toute l'orientation (par exemple la torsion de l'avant-bras 11 n'est pas donnée), pour cela il faut au moins un gyromètre des moyens 20. Dans la suite de la description de cette invention, il suffit que l'orientation de l'avant-bras 11 soit disponible ou même que l'angle d'élévation entre l'avant-bras 11 et la direction horizontale soit connu (avec précision).

*Couple contra gravitaire massique*

Dans une étape (b), les moyens de traitement de données 21, 31, 41 estiment un couple exercé par les muscles d'un bras 12 du membre supérieur 1 sur ledit avant-bras 11 en fonction de la vitesse angulaire mesurées, d'une orientation dudit avant-bras 11, et de paramètres physiques dudit avant-bras 11. Comme expliqué ce couple peut être un couple massique.

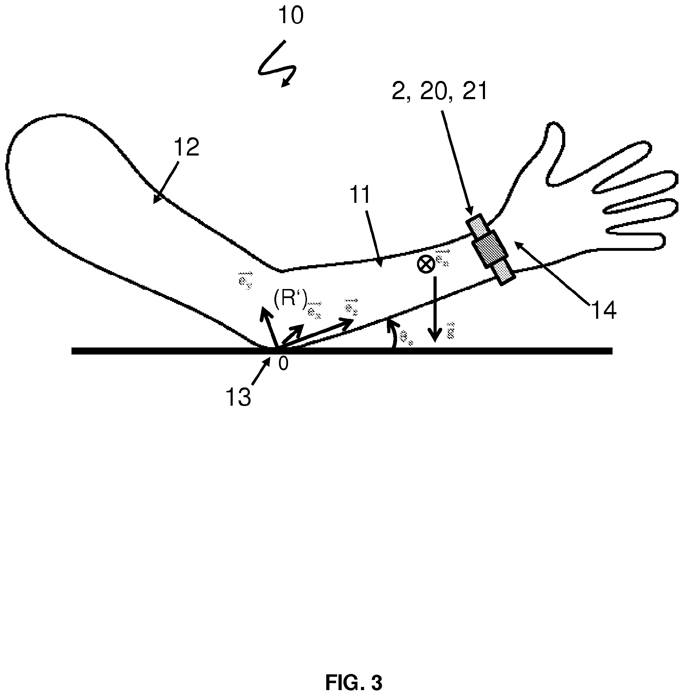

Pour cela, on remarque qu'en position assise, il est naturel d'avoir le coude 13 posé sur un support par exemple une table, un accoudoir, etc., comme représenté sur la **figure 3.** Une grande partie de l'activité consiste ensuite à déplacer l'avant-bras 11 dans un mouvement de rotation autour du point de contact entre le coude et le support. On peut noter que les personnes les plus affaiblies notamment du fait de troubles musculaires passent la majorité de leur temps assis en fauteuil avec le coude posé sur un support. Les personnes plus mobiles passent aussi beaucoup de temps avec les coudes posés sur un support par exemple un bureau, de plus il est possible d'identifier ces périodes à partir des mesures inertielles.

L'avant-bras 11 est modélisé comme un cylindre de longueur L, de rayon R et de masse volumique ρ, formant comme expliqué les paramètres physiques de l'avant-bras 11. On suppose que le coude 13 est posé sur l'accoudoir du fauteuil et on note O le point de contact. On considère les rotations de l'avant-bras 11 autour du point de contact. On utilise le référentiel ($R_O$) ayant pour origine O et dont les axes sont ceux d'un référentiel terrestre ainsi que le référentiel (R') ayant pour origine O et dont les axes sont liés à ceux de l'avant-bras 11. La vitesse angulaire $\vec{\omega}$ de l'avant-bras 11 est égal à la rotation de ($R_O$) par rapport à (R').

La vitesse d'un point de l'avant-bras 11 M dans ($R_O$) est donné par :

$$\vec{v_M} = \vec{\omega} \wedge \vec{OM}$$

Et par définition, le moment cinétique de l'avant-bras par rapport à O est:

$$\vec{L_O} = \iiint \vec{OM} \wedge \rho \, \vec{v_M} \, d\tau = \rho \iiint \vec{OM} \wedge \left(\vec{\omega} \wedge \vec{OM}\right) d\tau$$

En notant ρ la masse volumique et en intégrant sur le volume de l'avant-bras 11.

En coordonnées cylindriques ($\vec{e_r}$, $\vec{e_\theta}$, $\vec{e_z}$), avec $\vec{e_z}$ dans l'axe de l'avant-bras 11, cela donne:

$$\vec{L_O} = \rho \int_0^L \int_0^{2\cdot\pi} \int_0^R (r\,\vec{e_r} + z\,\vec{e_z}) \wedge \left(\vec{\omega} \wedge (r\,\vec{e_r} + z\,\vec{e_z})\right) r \, d_r \, d_\theta \, d_z$$

$$\vec{L_O} = \rho \int_0^L \int_0^{2\cdot\pi} \int_0^R \left((r^2 + z^2)\,\vec{\omega} - (\vec{\omega} \cdot (r\,\vec{e_r} + z\,\vec{e_z}))(r\,\vec{e_r} + z\,\vec{e_z})\right) r \, d_r \, d_\theta \, d_z$$

En utilisant $\vec{e_r}$ = cos θ $\vec{e_x}$ + sin θ $\vec{e_y}$ on obtient:

$$\vec{L_O} = \rho \int_0^L \int_0^{2\cdot\pi} \int_0^R \Big((r^2 + z^2)\,\vec{\omega}$$
$$- \left(\omega_x\,r\,\cos\theta + \omega_y\,r\,\sin\theta + \omega_z\,z\right)(r\,\cos\theta\,\vec{e_x} + r\,\sin\theta\,\vec{e_y}$$
$$+ z\,\vec{e_z})\Big) r \, d_r \, d_\theta \, d_z$$

En projetant sur l'axe $\vec{e_z}$

$$L_{Oz} = \rho \int_0^L \int_0^{2\cdot\pi} \int_0^R \left((r^2 + z^2)\,\omega_z - (\omega_x\,r\,\cos\theta + \omega_y\,r\,\sin\theta + \omega_z\,z)\,z\right) r \, d_r \, d_\theta \, d_z$$

$$L_{Oz} = \rho \int_0^L \int_0^{2\cdot\pi} \int_0^R \left(r^3\,\omega_z - \omega_x\,r^2\,z\,\cos\theta - \omega_y\,r^2\,z\,\sin\theta\right) d_r \, d_\theta \, d_z$$

Après intégration, il reste:

$$L_{Oz} = \frac{\rho \, 2 \, \pi \, L \, R^4 \, \omega_z}{4} = \frac{m \, R^2 \, \omega_z}{2}$$

De même en projetant sur l'axe $\vec{e_x}$

$$L_{Ox} = \rho \int_0^L \int_0^{2 \cdot \pi} \int_0^R \left( (r^2 + z^2) \, \omega_x - \left( r \, \omega_x \, \cos\theta + r \, \omega_y \, \sin\theta + \omega_z \, z \right) r \, \cos\theta \right) r \, d_r \, d_\theta \, d_z$$

$$L_{Ox} = \rho \int_0^L \int_0^{2 \cdot \pi} \int_0^R \left( (r^3 \, \sin^2\theta + r \, z^2) \, \omega_x - r^3 \cdot \omega_y \, \sin\theta \, \cos\theta - \omega_z \, r^2 \, z \, \cos\theta \right) d_r \, d_\theta \, d_z$$

Après intégration, il reste:

$$L_{Ox} = \rho \, \Omega_x \left( \frac{\pi \, L \, R^4}{4} + \frac{2 \, \pi \, L^3 \, R^2}{6} \right) = \left( \frac{R^2}{4} + \frac{L^2}{3} \right) m \, \omega_x$$

Par symétrie, le même calcul donne pour l'axe $\vec{e_y}$:

$$L_{Oy} = \left( \frac{R^2}{4} + \frac{L^2}{3} \right) m \, \omega_y$$

Dans le repère d'origine O et d'axes $(\vec{e_x}, \vec{e_y}, \vec{e_z})$ avec $\vec{e_z}$ dans l'axe de l'avant-bras 11 (voir figure 3), on peut donc écrire le tenseur d'inertie massique:

$$\bar{\bar{I}} = \begin{pmatrix} \dfrac{R^2}{4} + \dfrac{L^2}{3} & 0 & 0 \\ 0 & \dfrac{R^2}{4} + \dfrac{L^2}{3} & 0 \\ 0 & 0 & \dfrac{R^2}{2} \end{pmatrix}$$

$$\vec{L_O} = m \, \bar{\bar{I}} \, \vec{\omega}$$

Par définition, le moment des forces de gravitation sur l'avant-bras 11 par rapport à O s'écrit:

$$\vec{M_{PO}} = \iiint \vec{OM} \wedge \vec{g} \, \rho \, d\tau$$

En coordonnées cylindriques $(\vec{e_r}, \vec{e_\theta}, \vec{e_x})$ avec $\vec{e_z}$ dans l'axe de l'avant-bras 11, cela donne:

$$\vec{M_{PO}} = \left( \int_0^L \int_0^{2 \cdot \pi} \int_0^R (r \, \vec{e_r} + z \, \vec{e_z}) \, \rho \, r \, d_r \, d_\theta \, d_z \right) \wedge \vec{g}$$

$$\overrightarrow{M_{PO}} = \left( \rho \, \pi \, R^2 \int_0^L z \, dz \right) \overrightarrow{e_z} \wedge \vec{g} + \left( \rho \int_0^L \int_0^{2\cdot\pi} \int_0^R r^2 \, \overrightarrow{e_r} \, d_r \, d_\theta \, d_z \right) \wedge \vec{g}$$

Le second terme de la somme est nul (en utilisant un argument de symétrie) et on obtient après intégration et simplifications:

$$\overrightarrow{M_{PO}} = m \, \frac{L}{2} \, \overrightarrow{e_z} \wedge \vec{g}$$

En introduisant l'angle d'élévation $\theta_e$ et le vecteur unitaire $\overrightarrow{e_n}$ normal à l'avant-bras 11 et à la direction du champ de gravité tel que défini sur la figure, on a $\overrightarrow{e_z} \wedge \vec{g} = \cos \theta_e \, \overrightarrow{e_n}$

$$\overrightarrow{M_{PO}} = m \, \frac{L}{2} \, g \, \cos \theta_e \, \overrightarrow{e_n}$$

On utilise alors le théorème du moment cinétique en considérant que les seules forces appliquées à l'avant-bras sont son poids et les forces des muscles. En notant $\overrightarrow{M_{BrasO}}$ le moment des forces exercées par les muscles du bras 12 sur l'avant-bras 11, on a:

$$\frac{d\overrightarrow{L_O}}{dt} = \overrightarrow{M_{PO}} + \overrightarrow{M_{BrasO}}$$

$$\overrightarrow{M_{BrasO}} = \frac{d\overrightarrow{L_O}}{dt} - \overrightarrow{M_{PO}} = m \, \bar{\bar{I}} \, \frac{d\vec{\omega}}{dt} - m \, \frac{L}{2} \, g \, \overrightarrow{e_z} \wedge \vec{g}$$

Finalement, on note $\overrightarrow{C_M}$ le couple à exercer pour lever l'avant-bras 11 divisé par sa masse et on obtient:

$$\overrightarrow{C_M} = \bar{\bar{I}} \, \frac{d\vec{\omega}}{dt} - \frac{L}{2} \, g \, \cos \theta_e \, \overrightarrow{e_n}$$

$$\overrightarrow{C_M} = \begin{pmatrix} \dfrac{R^2}{4} + \dfrac{L^2}{3} & 0 & 0 \\ 0 & \dfrac{R^2}{4} + \dfrac{L^2}{3} & 0 \\ 0 & 0 & \dfrac{R^2}{2} \end{pmatrix} \times \begin{bmatrix} \dfrac{d\omega_x}{dt} \\ \dfrac{d\omega_y}{dt} \\ \dfrac{d\omega_z}{dt} \end{bmatrix} - \frac{L}{2} \, g \, \cos \theta_e \, \overrightarrow{e_n}$$

$$\overrightarrow{C_M} = \begin{bmatrix} \left( \dfrac{R^2}{4} + \dfrac{L^2}{3} \right) \dfrac{d\omega_x}{dt} \\ \left( \dfrac{R^2}{4} + \dfrac{L^2}{3} \right) \dfrac{d\omega_y}{dt} \\ \dfrac{R^2}{2} \dfrac{d\omega_z}{dt} \end{bmatrix} - \frac{L}{2} \, g \, \cos \theta_e \, \overrightarrow{e_n}$$

Comme expliqué, il est possible de négliger le terme portant sur le rayon devant le terme portant sur la longueur, et d'estimer le couple plus simplement par la formule :

$$\overrightarrow{C_M} = \begin{bmatrix} \dfrac{L^2}{3} \dfrac{d\omega_x}{dt} \\ \dfrac{L^2}{3} \dfrac{d\omega_y}{dt} \\ 0 \end{bmatrix} - \dfrac{L}{2}\, g\, \cos\theta_e\, \overrightarrow{e_n}$$

Cette formule peut être encore simplifiée en $\overrightarrow{C_M} = -\dfrac{L}{2}\, g\, \cos\theta_e\, \overrightarrow{e_n}$ en négligeant le terme portant sur la longueur, par exemple dans un mode de réalisation où l'on disposerait seulement d'un inclinomètre 24 et de moyens 20 capables de mesurer la vitesse angulaire (gyromètres), de sorte à calculer le couple uniquement à partir de l'angle d'élévation. Une telle valeur serait toutefois approximative et il apparait impératif de filtrer les erreurs de l'angle d'élévation avec les vitesses angulaires mesurées pour utiliser cette formule.

Dans une étape (c), les moyens de traitement de données 21, 31, 41 déterminent une puissance exercée par le membre supérieur 10 en fonction du couple estimé et de la vitesse angulaire mesurée.

Plus précisément la puissance (instantanée) est donnée par le produit scalaire du couple estimé et de la vitesse angulaire. En d'autres termes, elle est égale au couple multiplié par la vitesse angulaire. On peut le cas échéant de nouveau normaliser par la masse de l'avant-bras 11 et la puissance massique vaut:

$$P_M = \overrightarrow{C_M} \cdot \vec{\omega} = \bar{\bar{I}}\, \dfrac{d\vec{\omega}}{dt} \cdot \vec{\omega} - \dfrac{L}{2}\, g\, \cos\theta_e\, \overrightarrow{e_n} \cdot \vec{\omega}$$

$$P_M = \bar{\bar{I}}\, \dfrac{d\vec{\omega}}{dt} \cdot \vec{\omega} + \dfrac{L}{2}\, g\, \cos\theta_e\, \dfrac{d\theta_e}{dt}$$

Et en utilisant les projections sur les axes liés au boitier:

$$P_M = \begin{pmatrix} \dfrac{R^2}{4} + \dfrac{L^2}{3} & 0 & 0 \\ 0 & \dfrac{R^2}{4} + \dfrac{L^2}{3} & 0 \\ 0 & 0 & \dfrac{R^2}{2} \end{pmatrix} \dfrac{d\left(\omega_x{}^2 + \omega_y{}^2 + \omega_z{}^2\right)}{dt} + \dfrac{L}{2}\left(g_y\, \omega_x - g_x\, \omega_y\right)$$

Comme avant cette puissance peut être calculée plus facilement en négligeant R devant L, voire en négligeant L.

On remarque que dans tous les cas la puissance associée au couple exercé par le bras 12 sur l'avant-bras 11 est la somme de deux termes. Le premier terme vient de l'énergie cinétique permettant de mettre en mouvement l'avant-bras 11. Le second terme vient de l'énergie potentielle liée à la hauteur de l'avant-bras 11. On remarque que le second terme qui dérive d'une énergie potentielle peut s'exprimer uniquement à partir de l'angle d'élévation. Les dispositifs fournissant uniquement l'élévation peuvent donc tirer parti de cette méthode.

La puissance peut être de signe négatif lorsque l'on l'abaisse l'avant-bras 11 ou lorsque sa vitesse angulaire diminue. Même dans ces les muscles doivent fournir un effort pour retenir le membre. De façon préférée, l'étape (c) comprend l'identification du caractère moteur ou de retenue de la puissance déterminée, en particulier en fonction du signe de la vitesse angulaire et de l'orientation (fermeture du membre supérieur 10 pour un mouvement moteur, et ouverture du membre supérieur 10 pour un mouvement de retenue).

Plusieurs modèles sont envisageables pour estimer au mieux l'effort exercé par les muscles dans ce type de mouvement. On peut considérer uniquement les mouvements ayant une puissance positive car ce sont les mouvements demandant le plus d'énergie aux muscles. Ou bien on peut considérer la valeur absolue de la puissance en appliquant éventuellement un coefficient différent en fonction du signe de chaque terme constituant la puissance. Cela permet de refléter les différents types d'efforts effectués par les muscles.

La puissance peut être la grandeur demandée, et celle-ci peut être directement restituée/stockée par le boitier 2 ou un équipement autre tel que le terminal 3. On note que la puissance moyenne peut être calculée sur un parcours, le cas échéant la puissance moyenne des puissances positives, ou encore la moyenne de la valeur absolue des puissances.

Dans une étape (d), les moyens de traitement de données 21, 31, 41 peuvent déterminer une autre grandeur représentative de l'activité physique du membre supérieur 10 à partir de la puissance.

Par exemple peut être estimé le travail effectué par les muscles du bras lors de chaque mouvement à partir de la puissance du couple exercé par le bras 12 sur l'avant-bras 11, en sommant pour chaque échantillon la puissance massique pour connaitre le travail massique des muscles pendant la période considérée (le travail peut être négatif).

Alternativement, une énergie exercée par le membre supérieur sur un intervalle de temps en sommant la valeur absolue des puissances déterminées à chaque instant dudit intervalle de temps.

De façon préférée le procédé comprend en outre une étape (e) de comparaison de ladite grandeur physique estimée avec au moins un seuil prédéterminé de sorte à identifier un problème musculaire du membre supérieur 10 de la personne 1. Ce peut être un seuil minimal (par exemple, si le muscle de l'avant-bras n'est pas capable de dépasser une valeur de puissance massique, le patient peut souffrir d'une dégénérescence musculaire) mais également un seuil maximal (par exemple, des maladies de type chorées sont définies par la survenue de mouvements incontrôlables, brusques et irréguliers).

*Equipements et système*

Selon un deuxième aspect, l'invention concerne en particulier les équipements 2, 3, 4 pour la mise en œuvre de l'un ou l'autre des modes de réalisation du procédé.

Comme expliqué précédemment, selon un premier mode de réalisation l'équipement est un boitier 2 autonome comprenant les moyens de mesure inertielle 20 et les moyens de traitement de données 21 configurés pour la mise en œuvre des étapes du procédé.

Le boitier 2 comprend en outre des moyens d'attache 23 du boitier 2 au membre supérieur 10, et le cas échéant un magnétomètre 24, des moyens de stockage de données 22 (pour le stockage des accélérations/vitesse angulaires mesurées ou des grandeurs physiques estimées) et/ou des moyens de communication 25 pour l'exportation des résultats.

Selon un deuxième mode de réalisation, l'équipement est un terminal mobile 3 ou un serveur 4, adapté pour communiquer avec un boitier 2 comprenant les moyens de mesure inertielle 20. En d'autres termes, le terminal 3 ou le serveur 4 comprend les moyens de traitement 31 ou 41 configurés pour la mise en œuvre des étapes du procédé. Chaque boitier 2 peut tout de même comprendre des moyens de traitement de données 21 pour le contrôle des moyens 20 et la transmission (via des moyens de communication 25) des données mesurées aux moyens de traitement de données 31, 41.

Il est à noter que les moyens 21, 31, 41 peuvent le cas échéant se partager des étapes du procédé. Par exemple, en cas d'application médicale, les moyens de traitement 21 du boitier 2 peuvent réaliser les étapes jusqu'à (c), et a posteriori les moyens 41 du serveur 4 mettent en œuvre l'étape (d) de détermination d'une grandeur physique adéquate de sorte à identifier un éventuel trouble du mouvement de la personne 1.

L'invention concerne dans ce cas également le système comprenant l'équipement 3, 4 selon ce mode de réalisation et le ou les boitiers 2 « satellites » en connexion

Dans tous les cas, les moyens de traitement de données 21, 31, 41 de l'équipement 2, 3, 4 « principal » sont configurés pour mettre en œuvre :

- Un module de réception d'une vitesse angulaire d'un avant-bras 11 dudit membre supérieur 10 de ladite personne 1 (et le cas échéant de l'accélération de l'avant-bras 11) acquise(s) par des moyens de mesure inertielle 20 solidaires dudit avant-bras 10 ;
- Optionnellement, un module de détermination d'une orientation de l'avant-bras 11, en particulier en fonction et l'accélération et la vitesse angulaire mesurées ;
- Un module d'estimation d'un couple exercé par les muscles d'un bras 12 du membre supérieur 10 sur ledit avant-bras 11 en fonction de la vitesse angulaire mesurées, de l'orientation dudit avant-bras 11, et de paramètres physiques dudit avant-bras 11 selon la revendication 12 ;
- Un module de détermination d'une puissance exercée par le membre supérieur 10 en fonction du couple estimé et de la vitesse angulaire mesurée.

*Produit programme d'ordinateur*

Selon un troisième et un quatrième aspects, l'invention concerne un produit programme d'ordinateur comprenant des instructions de code pour l'exécution (sur les moyens de traitement 21, 31, 41) de l'activité physique exercée par un membre supérieur 10 d'une personne 1 selon la revendication 14, ainsi que des moyens de stockage lisibles par un équipement informatique (par exemple des moyens de stockage de données 22) sur lequel on trouve ce produit programme d'ordinateur selon la revendication 15.

**Revendications**

1. Procédé d'estimation de l'activité physique exercée par un membre supérieur (10) d'une personne (1), le procédé comprenant des étapes de :

   (a) Acquisition par des moyens de mesure inertielle (20) solidaires d'un avant-bras (11) dudit membre supérieur (10) de ladite personne (1) d'une vitesse angulaire dudit avant-bras (11) ;
   (b) Estimation par des moyens de traitement de données (21, 31, 41) d'un couple exercé par les muscles d'un bras (12) du membre supérieur (10) sur ledit avant-bras (11) en fonction de la vitesse angulaire mesurée, d'une orientation dudit avant-bras (11), et de paramètres physiques dudit avant-bras (11) ; ledit couple étant estimé comme la différence entre la dérivée d'un moment cinétique $\overrightarrow{L_O}$ de l'avant-bras (11) par rapport à un point O de contact du coude (13) de l'avant-bras (11) avec un support, et un moment $\overrightarrow{M_{PO}}$ des forces de gravitation sur l'avant-bras (11) par rapport au point O ; avec $\overrightarrow{L_O} = \iiint \overrightarrow{OM} \wedge (\vec{\omega} \wedge \overrightarrow{OM}) \, \rho \, d\tau$ et $\overrightarrow{M_{PO}} = \iiint \overrightarrow{OM} \wedge \vec{g} \, \rho \, d\tau$, où $\rho$ est la masse volumique, $\vec{\omega}$ est la vitesse angulaire mesurée et $\vec{g}$ l'accélération de la pesanteur, en intégrant en tout point M sur un volume de l'avant-bras (11) défini par lesdits paramètres physiques dudit avant-bras (11) ;
   (c) Détermination par les moyens de traitement de données (21, 31, 41) d'une puissance exercée par le membre supérieur (10) en fonction du couple estimé et de la vitesse angulaire mesurée.

2. Procédé selon la revendication 1, comprenant suite à l'étape (a) une étape (a1) de détermination de ladite orientation de l'avant-bras (11).

3. Procédé selon la revendication 2, dans lequel l'étape (a) comprend en outre l'acquisition par les moyens de mesure inertielle (20) d'une accélération dudit membre supérieur (10), l'orientation de l'avant-bras (11) étant déterminée en fonction et l'accélération et la vitesse angulaire mesurées.

4. Procédé selon la revendication 3, dans lequel la détermination de l'orientation de l'avant-bras (11) comprend la mise en œuvre d'un filtre estimateur d'état linéaire ou non linéaire.

5. Procédé selon l'une des revendications 1 à 4, dans lequel lesdits moyens de mesure inertielle (20) sont disposés sur ledit avant-bras (10) entre le coude (13) et un poignet (14).

6. Procédé selon l'une des revendications 1 à 5, dans lequel les paramètres physiques de l'avant-bras (11) comprennent une longueur de l'avant-bras (11) et un rayon de l'avant-bras (11), le couple estimé à l'étape (b) étant un couple par unité de masse dudit avant-bras (11) donné par la formule

$$\overrightarrow{C_M} = \begin{pmatrix} \frac{R^2}{4} + \frac{L^2}{3} & 0 & 0 \\ 0 & \frac{R^2}{4} + \frac{L^2}{3} & 0 \\ 0 & 0 & \frac{R^2}{2} \end{pmatrix} \times \begin{bmatrix} \frac{d\omega_x}{dt} \\ \frac{d\omega_y}{dt} \\ \frac{d\omega_z}{dt} \end{bmatrix} - \frac{L}{2} \, g \, \cos\theta_e \, \overrightarrow{e_n},$$

où $\vec{\omega}$ est la vitesse angulaire mesurée, $R$ le rayon de l'avant-bras (11), L la longueur de l'avant-bras (11), $\theta_e$ l'angle d'élévation de l'avant-bras (11) et $\overrightarrow{e_n}$ le vecteur unitaire normal à l'avant-bras (11) et à la direction de l'accélération de la pesanteur.

7. Procédé selon l'une des revendications 1 à 6, dans lequel la puissance exercée par le membre supérieur (10) est déterminée à l'étape (c) par le produit scalaire du couple estimé et de la vitesse angulaire mesurée.

8. Procédé selon l'une des revendications 1 à 7, dans lequel l'étape (c) comprend l'identification du caractère moteur ou de retenue de la puissance déterminée.

**9.** Procédé selon l'une des revendications 1 à 8, étant un procédé d'estimation d'une grandeur représentative de l'activité physique exercée par le membre supérieur (10) de la personne (1), le procédé comprenant une étape (d) d'estimation de ladite grandeur physique à partir de la puissance déterminée.

**10.** Procédé selon la revendication 9, dans lequel ladite grandeur physique est l'énergie exercée par le membre supérieur (10) sur un intervalle de temps, déterminée à l'étape (d) en sommant la valeur absolue des puissances déterminées à chaque instant dudit intervalle de temps.

**11.** Procédé selon l'une des revendications 9 et 10, comprenant en outre une étape (e) de comparaison de ladite grandeur physique estimée avec au moins un seuil prédéterminé de sorte à identifier un problème musculaire du membre supérieur (10) de la personne (1).

**12.** Equipement (2, 3, 4) pour la mise en œuvre d'un procédé de d'estimation de l'activité physique exercée par un membre supérieur (10) d'une personne (1), comprenant des moyens de traitement de données (21, 31, 41) configurés pour mettre en œuvre :

- Un module de réception d'une vitesse angulaire d'un avant-bras (11) dudit membre supérieur (10) de ladite personne (1) acquise par des moyens de mesure inertielle (20) solidaires dudit avant-bras (11) ;
- Un module d'estimation d'un couple exercé par les muscles d'un bras (12) du membre supérieur (10) sur ledit avant-bras (11) en fonction de la vitesse angulaire mesurée, d'une orientation dudit avant-bras (11), et de paramètres physiques dudit avant-bras (11) ; ledit couple étant estimé comme la différence entre la dérivée d'un moment cinétique $\overrightarrow{L_O}$ de l'avant-bras (11) par rapport à un point O de contact du coude de l'avant-bras (11) avec un support, et un moment $\overrightarrow{M_{PO}}$ des forces de gravitation sur l'avant-bras (11) par rapport au point O ; avec $\overrightarrow{L_O} = \iiint \overrightarrow{OM} \wedge (\vec{\omega} \wedge \overrightarrow{OM})\, \rho\, d\tau$ et $\overrightarrow{M_{PO}} = \iiint \overrightarrow{OM} \wedge g\, \rho\, d\tau$, où $\rho$ est la masse volumique, $\vec{\omega}$ est la vitesse angulaire mesurée et g le champ de pesanteur, en intégrant en tout point M sur un volume de l'avant-bras (11) défini par lesdits paramètres physiques dudit avant-bras (11) ;
- Un module de détermination d'une puissance exercée par le membre supérieur (10) en fonction du couple estimé et de la vitesse angulaire mesurée.

**13.** Equipement selon la revendication 12, étant soit un boitier (2) comprenant les moyens de mesure inertielle (20) et des moyens d'attache (23) du boitier (2) au membre supérieur (10) et des moyens de communication (25) ; soit un terminal mobile (3) ou un serveur (4), adapté pour communiquer avec un boitier (2) comprenant les moyens de mesure inertielle (20).

**14.** Produit programme d'ordinateur comprenant des instructions de code pour l'exécution de la part de l'équipement selon l'une des revendications 12 à 13 d'un procédé d'estimation de l'activité physique exercée par un membre supérieur (10) d'une personne (1) selon l'une des revendications 1 à 11, lorsque ledit programme est exécuté sur un ordinateur.

**15.** Moyen de stockage lisible par un équipement informatique sur lequel un produit programme d'ordinateur comprend des instructions de code pour l'exécution de la part de l'équipement selon l'une des revendications 12 à 13 d'un procédé d'estimation de l'activité physique exercée par un membre supérieur (10) d'une personne (1) selon l'une des revendications 1 à 11.

**Patentansprüche**

**1.** Verfahren zur Kalkulation der physischen Aktivität, die von einer oberen Gliedmaße (10) einer Person (1) ausgeübt wird, wobei das Verfahren die folgenden Schritte umfasst:

(a) Erfassen, durch Trägheitsmessmittel (20), die mit einem Unterarm (11) der oberen Gliedmaße (10) der Person (1) fest verbunden sind, einer Winkelgeschwindigkeit des Unterarms (11);
(b) Kalkulieren, durch Datenverarbeitungsmittel (21, 31, 41), eines Kraftmoments, das von den Muskeln eines Arms (12) der oberen Gliedmaße (10) auf den Unterarm (11) ausgeübt wird, in Abhängigkeit von der gemessenen Winkelgeschwindigkeit, einer Ausrichtung des Unterarms (11) und von physischen Parametern des Unterarms (11); wobei das Kraftmoment als die Differenz zwischen der Ableitung eines kinetischen Moments $\overrightarrow{L_o}$ des Unterarms (11) in Bezug auf einen Kontaktpunkt O des Ellbogens (13) des Unterarms (11) mit einer Unterlage

und einem Moment $\overrightarrow{M_{PO}}$ der Gravitationskräfte auf den Unterarm (11) in Bezug auf den Punkt O kalkuliert wird; mit $\overrightarrow{L_O} = \iiint \overrightarrow{OM}\,(\vec{\omega} \wedge \overrightarrow{OM})\,\rho\,d\tau$ und $\overrightarrow{M_{PO}} = \iiint \overrightarrow{OM} \wedge \vec{g}\,\rho\,d\tau$, wobei p die Volumenmasse ist, $\vec{\omega}$ die gemessene Winkelgeschwindigkeit ist und $\vec{g}$ die Beschleunigung der Schwerkraft ist, bei Integration an jedem Punkt M in ein Volumen des Unterarms (11), definiert durch die physischen Parameter des Unterarms (11);

(c) Bestimmen, durch die Datenverarbeitungsmittel (21, 31, 41), einer Leistung, die von der oberen Gliedmaße (10) in Abhängigkeit vom kalkulierten Kraftmoment und von der gemessenen Winkelgeschwindigkeit ausgeübt wird.

2. Verfahren nach Anspruch 1, das nach dem Schritt (a) einen Bestimmungsschritt (a1) der Ausrichtung des Unterarms (11) umfasst.

3. Verfahren nach Anspruch 2, wobei der Schritt (a) ferner die Erfassung, durch die Trägheitsmessmittel (20), einer Beschleunigung der oberen Gliedmaße (10) umfasst, wobei die Ausrichtung des Unterarms (11) in Abhängigkeit von der gemessenen Beschleunigung und der gemessenen Winkelgeschwindigkeit bestimmt wird.

4. Verfahren nach Anspruch 3, wobei die Bestimmung der Ausrichtung des Unterarms (11) die Anwendung eines linearen oder nicht linearen Zustandskalkulationsfilters umfasst.

5. Verfahren nach einem der Ansprüche 1 bis 4, wobei die Trägheitsmessmittel (20) auf dem Unterarm (10) zwischen dem Ellbogen (13) und einem Handgelenk (14) angeordnet sind.

6. Verfahren nach einem der Ansprüche 1 bis 5, wobei die physischen Parameter des Unterarms (11) eine Länge des Unterarms (11) und einen Radius des Unterarms (11) umfassen, wobei das in Schritt (b) kalkulierte Kraftmoment ein Kraftmoment je Masseneinheit des Unterarms (11) ist, berechnet nach der Formel

$$\overrightarrow{C_M} = \begin{pmatrix} \frac{R^2}{4} + \frac{L^2}{3} & 0 & 0 \\ 0 & \frac{R^2}{4} + \frac{L^2}{3} & 0 \\ 0 & 0 & \frac{R^2}{2} \end{pmatrix} \times \begin{bmatrix} \frac{d\omega_x}{dt} \\ \frac{d\omega_y}{dt} \\ \frac{d\omega_z}{dt} \end{bmatrix} - \frac{L}{2}\,g\,\cos\theta_e\,\overrightarrow{e_n}$$

wobei $\vec{\omega}$ die gemessene Winkelgeschwindigkeit ist, R der Radius des Unterarms (11), L die Länge des Unterarms (11), $\theta_e$ der Hebewinkel des Unterarms (11) und $\overrightarrow{e_n}$ der Normaleinheitsvektor zum Unterarm (11) und zur Richtung der Beschleunigung der Schwerkraft ist.

7. Verfahren nach einem der Ansprüche 1 bis 6, wobei die von der oberen Gliedmaße (10) ausgeübte Leistung in Schritt (c) durch das Skalarprodukt des kalkulierten Kraftmoments und von der gemessenen Winkelgeschwindigkeit bestimmt wird.

8. Verfahren nach einem der Ansprüche 1 bis 7, wobei der Schritt (c) die Identifikation des Antriebs- oder Rückhaltcharakters der bestimmten Leistung umfasst.

9. Verfahren nach einem der Ansprüche 1 bis 8, das ein Kalkulationsverfahren einer Größe ist, die für die physische Aktivität repräsentativ ist, die von der oberen Gliedmaße (10) der Person (1) ausgeübt wird, wobei das Verfahren einen Kalkulationsschritt (d) der physikalischen Größe auf der Basis der bestimmten Leistung umfasst.

10. Verfahren nach Anspruch 9, wobei die physikalische Größe die von der oberen Gliedmaße (10) in einem Zeitintervall ausgeübte Energie ist, die in Schritt (d) durch Summieren des absoluten Wertes der zu jedem Augenblick des Zeitintervalls bestimmten Leistungen bestimmt wurde.

11. Verfahren nach einem der Ansprüche 9 und 10, umfassend ferner einen Vergleichsschritt (e) der kalkulierten physischen Größe mit mindestens einem vorbestimmten Grenzwert, so dass ein Muskelproblem der oberen Gliedmaße (10) der Person (1) identifiziert wird.

12. Ausrüstung (2, 3, 4) zur Durchführung eines Verfahrens zur Kalkulation der physischen Aktivität, die von einer oberen Gliedmaße (10) einer Person (1) ausgeübt wird, die Datenverarbeitungsmittel (21, 31, 41) umfasst, die ausgelegt, um umzusetzen:

- ein Modul zum Empfangen einer Winkelgeschwindigkeit eines Unterarms (11) der oberen Gliedmaße (10) der Person (1), die von Trägheitsmessmitteln (20) erfasst wird, die mit dem Unterarm (11) fest verbunden sind;
- ein Modul zum Kalkulieren eines Kraftmoments, das von den Muskeln eines Arms (12) der oberen Gliedmaße (10) auf den Unterarm (11) ausgeübt wird, in Abhängigkeit von der gemessenen Winkelgeschwindigkeit, einer Ausrichtung des Unterarms (11) und von physischen Parametern des Unterarms (11); wobei das Kraftmoment als die Differenz zwischen der Ableitung eines kinetischen Moments $\overrightarrow{L_O}$ des Unterarms (11) in Bezug auf einen Kontaktpunkt O des Ellbogens des Unterarms (11) mit einer Unterlage und einem Moment $\overrightarrow{M_{PO}}$ der Gravitationskräfte auf den Unterarm (11) in Bezug auf den Punkt O kalkuliert wird; mit $\overrightarrow{L_O} = \iiint \overrightarrow{OM} \wedge (\vec{\omega} \wedge \overrightarrow{OM})\, \rho\, d\tau$ und $\overrightarrow{M_{PO}} = \iiint \overrightarrow{OM} \wedge \vec{g}\, \rho\, d\tau$, wobei p die Volumenmasse ist, $\vec{\omega}$ die gemessene Winkelgeschwindigkeit ist und $\vec{g}$ das Schwerefeld ist, bei Integration an jedem Punkt M in ein Volumen des Unterarms (11), definiert durch die physischen Parameter des Unterarms (11);
- ein Modul zum Bestimmen einer Leistung, die von der oberen Gliedmaße (10) in Abhängigkeit vom kalkulierten Moment und von der gemessenen Winkelgeschwindigkeit ausgeübt wird.

13. Ausrüstung nach Anspruch 12, die entweder ein Gehäuse (2) ist, umfassend die Trägheitsmessmittel (20) und Befestigungsmittel (23) des Gehäuses (2) an der oberen Gliedmaße (10) und Kommunikationsmittel (25); oder ein mobiles Endgerät (3) oder ein Server (4), das/der geeignet zur Kommunikation mit einem Gehäuse (2) ist, das die Trägheitsmessmittel (20) umfasst.

14. Rechnerprogrammprodukt, umfassend Codebefehle für die Ausführung, von der Ausrüstung nach einem der Ansprüche 12 bis 13, eines Verfahrens zur Kalkulation der physischen Aktivität, die von einer oberen Gliedmaße (10) einer Person (1) ausgeübt wird, nach einem der Ansprüche 1 bis 11, wenn das Programm auf einem Rechner ausgeführt wird.

15. Speichermittel, das von einer IT-Ausrüstung lesbar ist, auf welcher ein Rechnerprogrammprodukt Codebefehle zur Ausführung, von der Ausrüstung nach einem der Ansprüche 12 bis 13, eines Verfahrens zur Kalkulation der physischen Aktivität, die von einer oberen Gliedmaße (10) einer Person (1) ausgeübt wird, nach einem der Ansprüche 1 bis 11 umfasst.

## Claims

1. Method for estimating the physical activity exerted by an upper limb (10) of a person (1), the method comprising steps of:

    (a) Acquiring, by inertial measurement means (20) solidly attached to a forearm (11) of said upper limb (10) of said person (1), an angular speed of said forearm (11);
    (b) Estimating, by data processing means (21, 31, 41), a torque exerted by the muscles of an arm (12) of the upper limb (10) on said forearm (11) as a function of the measured angular speed, of an orientation of said forearm (11), and of physical parameters of said forearm (11); said torque being estimated as the difference between the derivative of a kinetic moment $\overrightarrow{L_O}$ of the forearm (11) with respect to a contact point O of the elbow (13) of the forearm (11) with a support, and a moment $\overrightarrow{M_{PO}}$ of the gravitational forces on the forearm (11) with respect to the point O; where $\overrightarrow{L_O} = \iiint \overrightarrow{OM} \wedge (\vec{\omega} \wedge \overrightarrow{OM})\, \rho\, d\tau$ and $\overrightarrow{M_{PO}} = \iiint \overrightarrow{OM} \wedge \vec{g}\, \rho\, d\tau$, where p is the density, $\vec{\omega}$ is the measured angular speed and $\vec{g}$ the acceleration due to gravity, by integrating at any point M on a volume of the forearm (11) defined by said physical parameters of said forearm (11);
    (c) Determining, by the data processing means (21, 31, 41), a power exerted by the upper limb (10) as a function of the estimated torque and of the measured angular speed.

2. Method according to claim 1, comprising after step (a) a step (a1) of determining said orientation of the forearm (11).

3. Method according to claim 2, wherein step (a) further comprises acquisition by the inertial measurement means (20) of an acceleration of said upper limb (10), the orientation of the forearm (11) being determined as a function of the measured acceleration and angular speed.

4. Method according to claim 3, wherein the determination of the orientation of the forearm (11) comprises the implementation of a linear or nonlinear state estimation filter.

5. Method according to one of claims 1 to 4, wherein said inertial measurement means (20) are arranged on said forearm (10) between the elbow (13) and a wrist (14).

6. Method according to one of claims 1 to 5, wherein the physical parameters of the forearm (11) comprise a length of the forearm (11) and a radius of the forearm (11), the torque estimated in step (b) being a torque per mass unit

$$\overrightarrow{C_M} = \begin{pmatrix} \frac{R^2}{4} + \frac{L^2}{3} & 0 & 0 \\ 0 & \frac{R^2}{4} + \frac{L^2}{3} & 0 \\ 0 & 0 & \frac{R^2}{2} \end{pmatrix} \times \begin{bmatrix} \frac{d\omega_x}{dt} \\ \frac{d\omega_y}{dt} \\ \frac{d\omega_z}{dt} \end{bmatrix} - \frac{L}{2} \, g \, \cos\theta_e \, \overrightarrow{e_n},$$

of said forearm (11) given by the formula
where $\vec{\omega}$ is the measured angular speed, R the radius of the forearm (11), L the length of the forearm (11), $\theta_e$ the angle of elevation of the forearm (11) and $\overrightarrow{e_n}$ the unit vector normal to the forearm (11) and to the direction of the acceleration due to gravity.

7. Method according to one of claims 1 to 6, wherein the power exerted by the upper limb (10) is determined in step (c) by the scalar product of the estimated torque and of the measured angular speed.

8. Method according to one of claims 1 to 7, wherein step (c) comprises the identification of the driving or resisting character of the determined power.

9. Method according to one of claims 1 to 8, being a method for estimating a quantity representative of the physical activity exerted by the upper limb (10) of the person (1), the method comprising a step (d) of estimating said physical quantity from the determined power.

10. Method according to claim 9, wherein said physical quantity is the energy exerted by the upper limb (10) over a time interval, determined in step (d) by adding together the absolute value of the powers determined at each instant of said time interval.

11. Method according to one of claims 9 and 10, further comprising a step (e) of comparing said estimated physical quantity with at least one predetermined threshold so as to identify a muscular problem of the upper limb (10) of the person (1).

12. Equipment (2, 3, 4) for the implementation of a method for estimating the physical activity exerted by an upper limb (10) of a person (1), comprising data processing means (21, 31, 41) configured to implement:

- A module for receiving an angular speed of a forearm (11) of said upper limb (10) of said person (1) acquired by inertial measurement means (20) solidly attached to said forearm (11);
- A module for estimating a torque exerted by the muscles of an arm (12) of the upper limb (10) on said forearm (11) as a function of the measured angular speed, of an orientation of said forearm (11), and of physical parameters of said forearm (11); said torque being estimated as the difference between the derivative of a kinetic moment $\overrightarrow{L_O}$ of the forearm (11) with respect to a contact point O of the elbow of the forearm (11) with a support, and a moment $\overrightarrow{M_{PO}}$ of the gravitational forces on the forearm (11) with respect to the point O; where $\overrightarrow{L_O} = \iiint \overrightarrow{OM} \wedge (\vec{\omega} \wedge \overrightarrow{OM}) \, \rho \, d\tau$ and $\overrightarrow{M_{PO}} = \iiint \overrightarrow{OM} \wedge \vec{g} \, \rho \, d\tau$, where $\rho$ is the density, $\vec{\omega}$ is the measured angular speed and $\vec{g}$ the field of gravity, by integrating at any point M on a volume of the forearm (11) defined by said physical parameters of said forearm (11);
- A module for determining a power exerted by the upper limb (10) as a function of the estimated torque and of the measured angular speed.

13. Equipment according to claim 12, being either a housing (2) comprising the inertial measurement means (20) and means (23) for attaching the housing (2) to the upper limb (10) and communication means (25); or a mobile terminal (3) or a server (4), adapted to communicate with a housing (2) comprising the inertial measurement means (20).

14. Computer program product comprising code instructions for the execution by the equipment according to one of claims 12 to 13 of a method for estimating the physical activity exerted by an upper limb (10) of a person (1) according to one of claims 1 to 11, when said program is executed on a computer.

15. Storage means readable by computer equipment on which a computer program product comprises code instructions for the execution by the equipment according to one of claims 12 to 13 of a method for estimating the physical activity exerted by an upper limb (10) of a person (1) according to one of claims 1 to 11.

**FIG. 1**

2

23

20

24

25

21 22

FIG. 2

FIG. 3

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1559591 A
- US 2015374283 A

- EP 2796123 A